Europäisches Patentamt

(19) European Patent Office

Office européen des brevets

(11) Numéro de publication: **0 181 272**
**B1**

(12) **FASCICULE DE BREVET EUROPEEN**

(45) Date de publication du fascicule du brevet: **22.11.90**

(21) Numéro de dépôt: **85420174.6**

(22) Date de dépôt: **02.10.85**

(51) Int. Cl.⁵: **A 61 M 1/36,** G 01 N 29/02

(54) **Détecteur de bulles dans le sang.**

(30) Priorité: **31.10.84 IT 5398784 u**

(43) Date de publication de la demande:
**14.05.86 Bulletin 86/20**

(45) Mention de la délivrance du brevet:
**22.11.90 Bulletin 90/47**

(84) Etats contractants désignés:
**BE CH DE FR GB IT LI NL SE**

(56) Documents cités:
**FR-A-2 198 640**

(73) Titulaire: **HOSPAL AG**
**Missionsstrasse 60/62**
**CH-4012 Basel (CH)**

(72) Inventeur: **Aldrovandi, Mauro**
**Via Statale Nord 7/B**
**I-41037 Mirandola Modena (IT)**
Inventeur: **Facchini, Mauro**
**51 A, Via Mameli**
**Mirandola (IT)**

(74) Mandataire: **Gauckler, Jacques**
**HOSPAL C.O.T. SCE Brevets B.P. 21 13, Avenue**
**de Lattre de Tassigny**
**F-69881 Meyzieu Cédex (FR)**

**Description**

La présente invention concerne un dispositif détecteur de la présence d'un fluide gazeux dans un liquide à l'intérieur d'une enceinte au-dessous d'un niveau prédéterminé. Cette invention concerne plus particulièrement un dispositif utilisant comme moyen de détection de la présence d'un fluide gazeux des transducteurs susceptibles de transmettre et de recevoir un signal sensible à la présence d'un fluide gazeux. Pour cela on préfère utiliser des transducteurs piézoélectriques aptes à transmettre et à recevoir des signaux à fréquence ultrasonique, dont la propagation est fortement influencée par la présence, même en très faibles quantités, d'un fluide gazeux.

Afin d'éviter l'emprisonnement d'air entre les transducteurs et les surfaces opposées correspondantes de l'enceinte dans laquelle on doit effectuer la mesure, il est connu, par exemple par le FR 2 198 640, d'utiliser des moyens élastiques qui poussent les transducteurs contre les surfaces respectives opposées de l'enceinte. Ces moyens sont en général constitués par des ressorts hélicoïdaux logés à l'intérieur d'un corps supportant ladite enceinte, qui poussent élastiquement des éléments supports desdits transducteurs le long de glissières ménagées dans le corps de l'appareil. Dans une variante de réalisation, l'un des transducteurs est fixé au corps de l'appareil par un ressort hélicoïdal.

Si les dispositifs connus mentionnés ci-dessus permettent effectivement de détecter la présence de fluide gazeux avec bonnes précision et fiabilité, ils sont particulièrement coûteux, essentiellement par le fait qu'ils exigent la réalisation de guides très précis pour les éléments support des transducteurs.

En outre, l'utilisation d'un dispositif détecteur, dans lequel un seul des transducteurs est amovible, rend les opérations de préparation de la mesure plus complexes, puisqu'il est nécessaire de maintenir le ressort tendu d'une main et de placer correctement l'enceinte entre les deux transducteurs de l'autre main.

Le but de la présente invention est de réaliser un dispositif de détection qui permette de surmonter les inconvénients liés aux dispositifs connus des types mentionnés ci-dessus et en particulier un dispositif qui ne nécessite pas l'exécution de guides précis pour le support des transducteurs et qui soit en outre facile à utiliser.

Ce but est atteint par la présente invention qui concerne un dispositif détecteur soit de la présence de gaz dans un liquide contenu dans une enceinte déformable et élastique, soit de l'abaissement du niveau d'un tel liquide en dessous d'une ligne prédéterminée à l'intérieur de l'enceinte, comprenant:

un corps creux présentant un logement capable de recevoir une partie de ladite enceinte dans une position prédisposée par rapport à ladite ligne prédéterminée,

une paire de transducteurs, respectivement transmetteur et récepteur d'un signal prédéterminé, fixés au dit corps creux en positions diamétralement opposées se faisant face, tournés vers ledit logement; ledit transducteur-transmetteur étant capable de coopérer avec ledit transducteur-transmetteur et à émettre un signal sensible à la quantité de fluide gazeux interposée entre lesdits transducteurs,

caractérisé en ce qu'il comporte en outre un élément mobile relié au dit corps creux par des moyens élastiques et susceptible de transmettre une poussée radiale à ladite partie de l'enceinte capable de la déformer et de provoquer ainsi son adhésion en des zônes opposées contre les transducteurs sans inclusion d'air.

Pour mieux comprendre la présente invention, on va maintenant décrire une forme préférée de réalisation à titre d'exemple non limitatif et en référence aux dessins ci-joints dans lesquels:

La figure 1 est la vue en élévation d'un dispositif de détection disposé autour d'une enceinte et illustré dans une position où il est prêt pour une mesure.

La figure 2 représente le dispositif selon la figure 1 disposé pour détecter un fluide gazeux à l'intérieur de ladite enceinte.

La figure 3 est une vue en coupe selon le plan III, III de la figure 2.

La figure 4 est une vue en coupe selon le plan IV, IV de la figure 3.

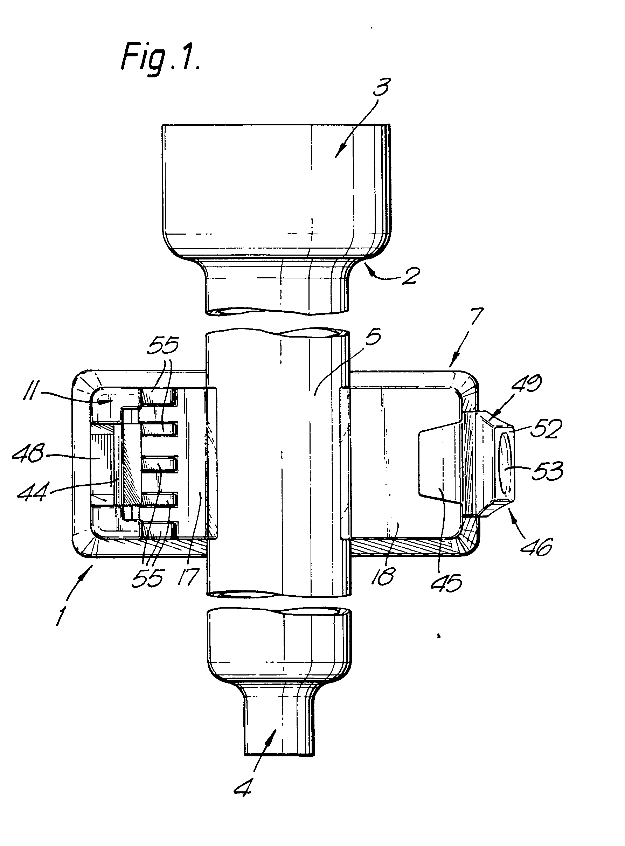

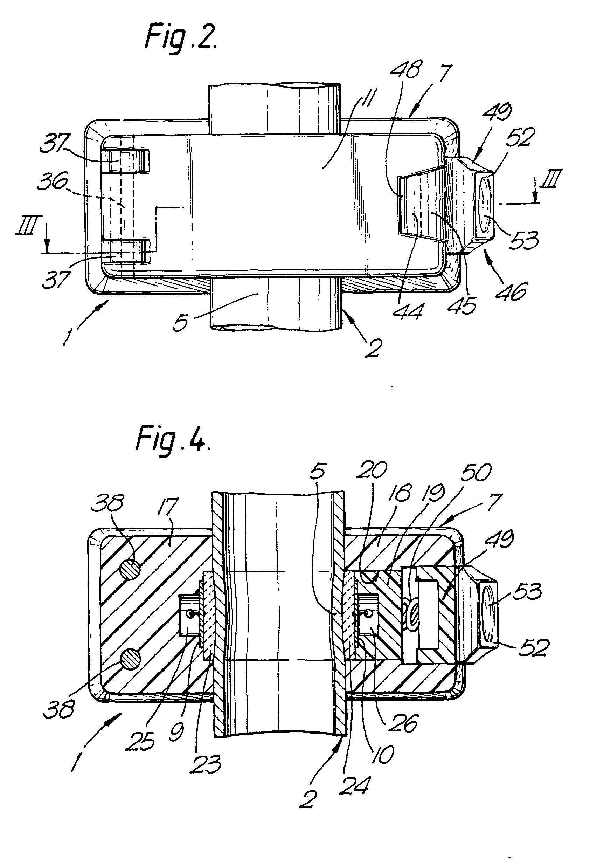

En se référant particulièrement aux figures 1 et 2, le repère 1 désigne l'ensemble d'un dispositif détecteur de la présence d'un fluide gazeux en relation avec le niveau prédéterminé du liquide dans une enceinte 2. L'enceinte 2 est essentiellement de type tubulaire avec des parois partiellement déformables de manière élastique. Elle présente respectivement une ouverture supérieure 3 élargie pour l'entrée d'un liquide et une ouverture inférieure 4 qui se rétrécit pour la sortie de ce même liquide. Advantageusement l'enceinte 2 pourrait être incorporée le long d'un circuit extra-corporel de sang, auquel cas le liquide à l'intérieur duquel on désirerait détecter la présence de fluide gazeux serait le sang.

Le dispositif 1 est capable de recevoir la partie 5 de l'enceinte 2 placée en position intermédiaire entre les ouvertures supérieure (3) et inférieure (4) de l'enceinte (2) elle-même.

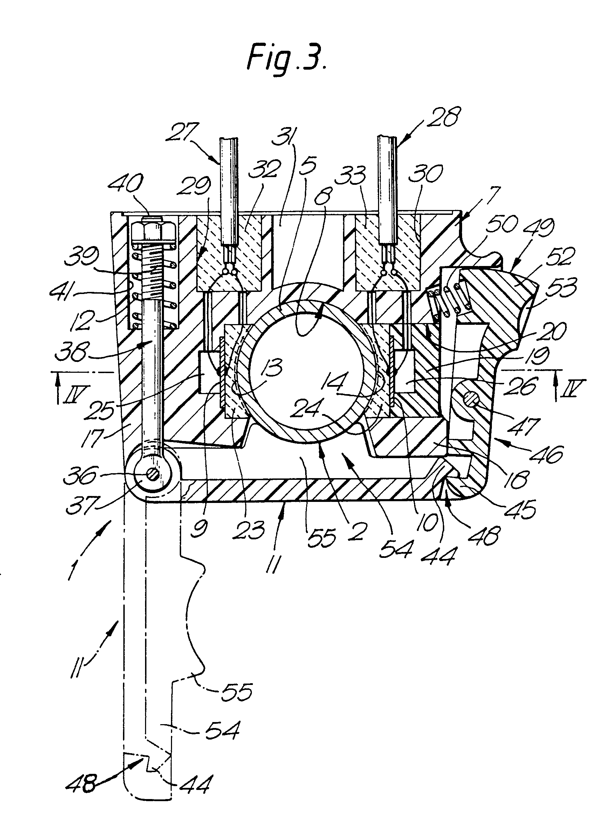

En se référant plus particulièrement à la figure 3, le dispositif 1 comprend essentiellement:

un corps creux 7 présentant un logement 8 susceptible de recevoir la partie intermédiaire 5 de l'enceinte 2.

une paire de transducteurs 9, 10 de type piézoélectrique, utilisés respectivement comme transmetteur et récepteur d'un signal à fréquence ultrasonique, fixés sur le corps 7 en positions diamétralement opposées et se faisant face, tournés vers le logement 8; et

un couvercle 11 relié au corps 7 par un ressort hélicoïdal 12 selon des dispositions décrites ci-après plus en détail et dont la fonction essentielle est de transmettre une poussée radiale sur la partie 5 de l'enceinte 2 ayant pour but d'entraîner une déformation et en conséquence l'adhésion des faces opposées 13, 14 de la partie 5 sur les transducteurs 9 et 10 sans emprisonnement d'air entre les faces en contact.

En examinant plus en détail le dispositif 1, on observe que le corps creux 7 a essentiellement la forme d'un U et qu'il supporte les transducteurs 9 et 10 resepctivement à l'aide de bras 17 et 18. En particulier le transducteur 9 est supporté directement par le bras 17, pendant que le transducteur 10 est supporté par un élément 19 emmanché de force à l'interieur d'un trou transversal 20 que présente le bras 18.

Le logement 8 est construit de manière à être légèrement élliqtique, le grand axe de l'ellipse étant confondu avec l'axe de symétrie qui relie les transducteurs 9 et 10. Sur les côtés tournés vers le logement 8, ces transducteurs sont recouverts d'une couche 23, 24 de matière plastique, telle que par exemple une résine époxy. En se reportant aux figures 3 et 4, on observe que la surface libre de chaque couche 23, 24 prend la configuration d'une selle de manière à créer une légère restriction de la partie du logement 8 en contact avec la partie 5 dans laquelle on désire détecter la présence de fluide gazeux.

Du coté opposé au logement 8 de chaque transducteur, se trouvent deux chambres 25, 26 qui permettent aux transducteurs piézoélectriques 9 et 10 de vibrer. Les faces opposées de chaque transducteur 9, 10 sont reliées par des fils conducteurs fins à des câbles électriques 27, 28; en particulier la jonction entre les fils conducteurs et les câbles 27 et 28 a lieu à l'intérieur des chambres respectives 29 et 30, ménagées dans la zône intermédiaire 31 du corps en U (7) et elles sont avantageusement remplies avec une résine durcissable 32, 33 en une matière plastique convenable.

En se reportant aux figures 2, 3 et 4, on observe que le couvercle 11 a une forme essentiellement rectangulaire et qu'il peut tourner autour d'un axe 36 (figures 2 et 3) supporté par les extrémités 37 d'une paire de tiges 38, dont chacune peut coulisser à l'intérieur d'un logement longitudinal à l'intérieur du bras 17. Chaque tige 38 présente, du coté opposé à l'extrémité 37, un tronçon fileté 39 (voir la figure 3) sur lequel se visse un écrou 40. Le logement de chaque tige 38 s'élargit essentiellement à hauteur de tronçon 39, constituant une chambre cylindrique 41 qui contient le ressort hélicoïdal 12 déjà mentionné, coaxial à la tige 38 et taré par serrage de l'écrou 40.

Du coté opposé à celui tournant autour de l'axe 36 le couvercle 11 présente un crochet 44 susceptible de coopérer avec un crochet correspondant 45 disposé à l'extrémité d'un bras 46 susceptible de pivoter autour d'un axe 47 fixé transversalement sur le bras 18. En se référant à la figure 2, on voit que le crochet 45 du bras pivotant 46 est essentiellement de forme trapézoïdale et se rétrécit vers l'extrémité, alors que le crochet 44 est situé au dessous du logement 48, aussi de forme trapézoïdale et complémentaire à celle du crochet 45, afin de faciliter le verrouillage du crochet 45 sur le crochet 44.

Du coté opposé à celui qui comporte le crochet 45, le bras pivotant 46 présente une extrémité 49 qui délimite une partie du logement d'un ressort hélicoïdal 50. Un tel ressort est compris entre l'extrémité 49 du bras pivotant 46 et la surface opposée du bras 18, tournée vers l'extérieur et transmettant au bras pivotant 49 une poussée élastique susceptible de faire tourner celui-ci dans le sens des aiguilles d'une montre (voir la figure 3) afin de faciliter l'accrochage entre les crochets 44 du couvercle 11 et 45 du bras pivotant 46.

Du coté opposé à celui du ressort 50, l'extrémité 49 présente un renflement 52 formant essentiellement un tronc de pyramide sur la surface de laquelle est ménagé un emplacement creux circulaire, concave 53.

On observe enfin, en se référant aux figures 1 et 3 que le couvercle 11 présente du coté tourné vers le logement 8 du corps creux 7, une pluralité de nervures longitudinales parallèles 54, qui s'étendent chacune essentiellement de l'extrémité située vers l'axe 36 jusqu'à l'extrémité opposée munie du crochet 44. Dans la partie faisant face au logement 8, chaque nervure 54 présente un profil constituant essentiellement un arc d'éllipse pour compléter et fermer le logement 8.

Le dispositif 1 est utilisé de la manière suivante:

Avant tout l'enceinte (2) est introduite dans le logement 8, comme représenté figure 1. Puis on ferme le couvercle 11 en le poussant jusqu'à ce que le crochet 45 se verrouille sur le crochet 44 (voir figure 2). Dans ces conditions le couvercle 11 est maintenu contre le corps creux 7 d'un coté par le crochet 44 et du coté opposé par l'axe 36 et les tiges 38.

Puisque celles-ci sont soumises à des efforts de traction axiaux élastiques sous l'effet des ressorts 12, il en résulte que le couvercle 11 transmet à la partie 5 de l'enceinte 2 un effort de compression radial par l'intermédiaire des profils elliptiques 55 des nervures longitudinales 54. De telles forces radiales entraînent la déformation de la partie 5 comportant les zones opposées 13, 14 de manière à les faire adhérer parfaitement, sans emprisonner d'air, sur les faces opposées 23, 24 des transducteurs 9 et 10. La déformation de la partie 5 de l'enceinte 2 dans la direction des transducteurs 9 et 10 est favorisée par le fait que le logement 8 est partiellement élliptique et présente, comme déjà mentionné, le grand axe dans la direction des transducteurs.

Dans ces conditions, le dispositif (1) est prêt à être utilisé comme détecteur de la présence d'un fluide gazeux à l'intérieur de la partie (5) de l'enceinte (2). A ce sujet il est suffisant d'alimenter le transducteur-transmetteur 9 avec un signal de fréquence prédéterminée, par exemple ultrasonique, et de relever le signal correspondant émis par le transducteur-récepteur 10. Puisque, comme on le sait, l'amplitude du signal émis par le transducteur 10 dépend de la présence de fluide gazeux, par exemple de très fines bulles d'air, entre les transducteurs 9 et 10, il sera suffisant de comparer la valeur effective d'une telle amplitude avec une valeur de référence pour détecter la présence éventuelle de fluide gazeux à hauteur de la zône intermédiaire (5).

Il est évident que le dispositif 1 permet de détecter soit la présence de fluide gazeux dans le liquide de l'enceinte (2), soit un abaissement du niveau d'un tel liquide en dessous de la zône placée sous le contrôle des transducteurs 9 et 10.

Dans le cas où l'on souhaite retirer l'enceinte 2, il suffira d'appuyer avec un doigt sur l'emplacement circulaire creux 53 à l'extrémité 49 du bras pivotant 46 (figure 3). Ce geste entraîne la rotation dans le sens inverse des aiguille d'une montre du bras pivotant 46 autour de l'axe 47 et compresse le ressort 50 jusqu'à ce que le crochet 45 libère le crochet 44 du couvercle 11. Celui-ci tourne partiellement autour de l'axe 36 sous l'effet de la réaction élastique exercée par la partie 5 de l'enceinte 2, puis il peut être ouvert complètement par l'opérateur jusqu'à la position indiquée en pointillés figure 3.

L'examen des caractéristiques du dispositif détecteur réalisé selon la présente invention permet de mettre en évidence les avantages obtenus. Tout particulièrement la réalisation de guides précis pour les éléments support des transducteurs piézoélectriques n'est pas nécessaire puisqu'ils sont fixés sur le corps creux 7. Celui-ci peut être moulé par injection, ce qui contribue à réduire le coût du dispositif 1. D'autre part, les opérations à effectuer pour disposer l'enceinte 2 dans le logement 8 puis pour fermer le couvercle 11 sont élémentaires et peuvent être facilement exécutées d'une seule main.

Enfin il est clair que diverses modifications et variantes de réalisation peuvent être apportées par le technicien au dispositif 1 décrit ci-avant sous pour cela sortir du cadre de la présente invention, tel que défini par les revendications suivantes.

**Revendications**

1. Dispositif (1) détecteur soit de la présence de gaz dans un liquide contenu dans une enceinte (2) déformable et élastique, soit de l'abaissement du niveau d'un tel liquide en dessous d'une ligne prédéterminée à l'intérieur de l'enceinte, comprenant:

un corps creux (7) présentant un logement capable de recevoir une partie (5) de ladite enceinte dans une position prédisposée par rapport à ladite ligne prédéterminée,

une paire de transducteurs, respectivement transmetteur (9) et récepteur (10) d'un signal prédéterminé, fixés au dit corps creux en positions diamétralement opposées se faisant face, tournés vers ledit logement (8); ledit transducteur-récepteur (10) étant capable de coopérer avec ledit transducteur-transmetteur (9) et à émettre un signal sensible à la quantité de fluide gazeux interposée entre lesdits transducteurs (9, 10),

caractérisé en ce qu'il comporte en outre un élément mobile (11) relié au dit corps creux (7) par des moyens élastiques (12) et susceptible de transmettre une poussée radiale à ladite partie (5) de l'enceinte (2), capable de la déformer et de provoquer ainsi son adhésion en des zônes opposées (13, 14), contre les transducteurs (9, 10) sans inclusion d'air.

2. Dispositif selon la revendication 1, caractérisé par le fait que ledit corps creux (7) est essentiellement en forme de U et porte les transducteurs-transmetteurs 9 et récepteur 10 sur les bras opposés (17, 18), l'élément mobile (11) étant capable de fermer ladite enceinte (2) à l'intérieur dudit corps creux (7).

3. Dispositif selon la revendication 2, caractérisé par le fait que ledit élément mobile (11) est constitué essentiellement par un couvercle (11) pivotant autour d'un axe 36 situé à l'extrémité du bras 17 dudit corps creux 7 et susceptible d'être accroché à l'extrémité de l'autre bras 18 par des moyens d'accrochage 46.

4. Dispositif selon la revendication 3, caractérisé par le fait que ledit axe 36 est supporté d'une manière qui lui permet de coulisser le long dudit premier bras 17, grâce à l'aide d'au moins une tige 38 mobile axialement contre l'action desdits moyens élastiques 12.

5. Dispositif selon la revendication 4, caractérisé par le fait que les moyens élastiques (12) comprennent au moins un ressort hélicoïdal coaxial à la tige 38 et susceptible d'exercer sur l'axe 36 une poussée élastique tendant à rapprocher ledit axe 36 et, avec celui-ci, ledit couvercle 11 des surfaces en regard desdits premier et second bras (17, 18) dudit corps 7 en U.

6. Dispositif selon la revendication 5, caractérisé par le fait que ledit ressort 12 est logé à l'intérieur d'une chambre cylindrique (41) ménagée à la base dudit premier bras 17.

7. Dispositif selon l'une quelconque des revendications 3 à 6, caractérisé par le fait que lesdits moyens d'accrochage sont constitués essentiellement par un bras pivotant 46 muni à une extrémité d'un crochet 45 susceptible d'engager une partie 44 correspondante à l'extrémité dudit couvercle 11.

8. Dispositif selon la revendication 7, caractérisé par le fait que ledit crochet 45 dudit bras pivotant 46 se rétrécit et est susceptible de s'engager dans un logement rétréci 48 présenté vers l'extrémité dudit couvercle 11.

9. Dispositif selon la revendication 8, caractérisé par le fait que ledit crochet 45 et ledit logement 48 ont essentiellement des formes trapézoïdales.

10. Dispositif selon l'une quelconque des revendications 7 à 9, caractérisé par le fait que ledit bras 46 pivote autour d'un axe 47 porté par ledit second bras (18) dudit corps creux 7 en forme de U.

11. Dispositif selon la revendication 10, caractérisé par le fait que ledit bras 46 pivote autour de l'axe 47 contre l'action des moyens élastiques 50.

12. Dispositif selon la revendication 11, caractérisé par le fait que lesdits moyens élastiques 50 sont essentiellement interposés entre l'extrémité 49 dudit bras pivotant 46 qui est à l'opposé du crochet 45 d'une part et la surface en regard dudit second bras (18) d'autre part.

13. Dispositif selon la revendication 12, caractérisé par le fait que ladite extrémité 49 du bras pivotant présente un bossage 52 s'étendant vers l'extérieur et muni d'une cuvette 53 délimitant une zône de commande de la rotation du bras pivotant 46.

14. Dispositif selon l'une quelconque des revendications précédentes, caractérisé par le fait que la surface de chaque transducteur, transmetteur 9 et récepteur 10, dirigée vers le logement 8, est recouverte d'une couche (23, 24) de matériau protecteur.

15. Dispositif selon la revendication 14, caractérisé par le fait que le matériau en question est de la résine époxy.

16. Dispositif selon l'une quelconque des revendications précédentes, caractérisé par le fait que chaque transducteur (9, 10) est de type piézoélectrique et que du coté opposé à celui dirigé vers le logement 8, il est face à une chambre (25, 26) qui permet à chacun desdits transducteurs de vibrer.

17. Dispositif selon la revendication 16, caractérisé par le fait que l'une (25) desdites chambres (25, 26) qui fait face au transducteur (9) est ménagée à l'intérieur dudit premier bras (17) dudit corps en U, tandis que l'autre chambre (26), qui fait face à l'autre transducteur (10) est ménagée dans un élément support (19), portant ledit transducteur (10), fixé au dit second bras 18 dudit corps (7) en U.

18. Dispositif selon la revendication 17, caractérisé par le fait que ledit élément (19) support dudit transducteur 10 est emmanché de force dans un trou (20) transversal au dit second bras 18 dudit corps (7) en U.

19. Dispositif selon l'une quelconque des revendications précédentes, caractérisé par le fait que ledit logement (8) a une section transversale elliptique dont le grand axe coïncide avec l'axe de symétrie commun aux dits transducteurs transmetteur (9) et récepteur (10).

**Patentansprüche**

1. Vorrichtung (1) zum Nachweis entweder der Anwesenheit von Gas in einer Flüssigkeit, die in einem verformbaren und elastischen Behältnis (2) enthalten ist, oder der Verminderung des Niveaus einer solchen Flüssigkeit unterhalb einer vorbestimmten Linie im Inneren des Behältnisses, bestehend aus:

einem Hohlkörper (7), der eine Lagerung aufweist, die einen Teil (5) des genannten Behältnisses in einer vorbestimmten Stellung in Bezug auf die genannte vorbestimmte Linie aufzunehmen vermag,

einem Paar Wandler, Geber (9) und Empfänger (10) eines vorbestimmten Signals, die an dem Hohlkörper in diametral mit der Vorderseite gegenüberliegenden Stellungen der Lagerung (8) zugewendet fixiert sind, wobei der Wandler-Empfänger (10) in der Lage ist, mit dem Wandler-Geber (9) zusammenzuarbeiten und ein Signal auszusenden, das auf die Menge des gasförmigen Fluids zwischen den Wandlern (9, 10) anspricht, dadurch gekennzeichnet,

daß sie außerdem ein bewegliches Element (11) aufweist, das mit dem Hohlkörper (7) durch elastische Mittel (12) verbunden und in der Lage ist, auf den Teil (5) des Behältnisses (2) einen radialen Schub zu übertragen, der imstande ist, es zu deformieren und so seine Haftung in gegenüberliegenden Zonen (13, 14) gegen die Wandler (9, 10) ohne Einschluß von Luft hervorzurufen.

2. Vorrichtung gemäß Anspruch 1, dadurch gekennzeichnet, daß der Hohlkörper (7) im wesentlichen in U-Form ist und die Wandler-Geber (9) und -Empfänger (10) auf gegenüberliegenden Armen (17, 18) trägt, wobei das bewegliche Element (11) imstande ist, das Behältnis (2) im Innern dieses Hohlkörpers (7) zu schließen.

3. Vorrichtung gemäß Anspruch 2, dadurch gekennzeichnet, daß das bewegliche Element (11) im wesentlichen aus einem Deckel (11) besteht, der um eine Achse (36), die am Ende des Armes (17) des Hohlkörpers (7) angeordnet ist, schwenkbar ist und in der Lage ist, am Ende des anderen Armes (18) durch Rastmittel (46) eingehakt zu werden.

4. Vorrichtung gemäß Anspruch 3, dadurch gekennzeichnet, daß die Achse (36) derart angebracht ist, daß sie entlang des ersten Armes (17) mit Hilfe wenigstens einer Stange (38), die axial gegen die Wirkung der elastischen Mittel (12) beweglich ist, verschoben werden kann.

5. Vorrichtung gemäß Anspruch 4, dadurch gekennzeichnet, daß die elastischen Mittel (12) wenigstens eine schraubenförmige Feder umfassen, die zur Stange (38) koaxial und in der Lage ist, auf die Achse (36) einen elastischen Schub auszuüben, um die Achse (36) und mit ihr den Deckel (11) den Flächen gegenüber dem ersten und zweiten Arm (17, 18) des U-förmigen Körpers (7) zu nähern.

6. Vorrichtung gemäß Anspruch 5, dadurch gekennzeichnet, daß die Feder (12) im Innern einer zylindrischen Kammer (41) gelagert ist, die an der Basis des ersten Arms (17) ausgebildet ist.

7. Vorrichtung gemäß einem der Ansprüche 3 bis 6, dadurch gekennzeichnet, daß die Rastmittel im wesentlichen aus einem schwenkbaren Arm (46) bestehen, der an einem Ende mit einem Haken (45) versehen ist, der in einen entsprechenden Teil (44) am Ende des Deckels (11) eingreifen kann.

8. Vorrichtung gemäß Anspruch 7, dadurch gekennzeichnet, daß der Haken (45) des schwenkbaren Arms (46) sich verjüngt und in der Lage ist, in eine verengte Lagerstelle (48) einzugreifen, die am Ende des Deckels (11) vorgesehen ist.

9. Vorrichtung gemäß Anspruch 8, dadurch gekennzeichnet, daß der Haken (45) und die Lagerung (48) im wesentlichen trapezoide Formen haben.

10. Vorrichtung gemäß einem der Ansprüche 7 bis 9, dadurch gekennzeichnet, daß der Arm (46) um eine Achse (47) schwenkbar ist, die durch den zweiten Arm (18) des U-förmigen Hohlkörpers (7) getragen wird.

11. Vorrichtung gemäß Anspruch 10, dadurch gekennzeichnet, daß der Arm (46) um die Achse (47) gegen die Wirkung elastischer Mittel (50) verschwenkbar ist.

12. Vorrichtung gemäß Anspruch 11, dadurch gekennzeichnet, daß die elastischen Mittel (50) im wesentlichen an dem Ende (49) des schwenkbaren Arms (46) angeordnet sind, das einerseits auf der entgegengesetzten Seite des Hakens (45) und andererseits auf der Vorderseite gegenüber dem zweiten Arm (18) angeordnet ist.

13. Vorrichtung gemäß Anspruch 12, dadurch gekennzeichnet, daß das Ende (49) des schwenkbaren Armes eine Erhöhung (52) aufweist, welche sich nach außen erstreckt und mit einer Mulde (53) versehen ist, die eine Bedienungszone der Drehung des schwenkbaren Armes (46) begrenzt.

14. Vorrichtung gemäß einem der vorhergehenden Ansprüche, dadurch gekennzeichnet, daß die Oberfläche jedes Wandlers, Geber (9) und Empfänger (10), die gegen die Lagerung (8) gerichtet ist, mit einer Schicht (23, 24) von Schutzmaterial bedeckt ist.

15. Vorrichtung gemäß Anspruch 14, dadurch gekennzeichnet, daß das in Frage stehende Material Epoxyharz ist.

16. Vorrichtung gemäß einem der vorhergehenden Ansprüche, dadurch gekennzeichnet, daß jeder Wandler (9, 10) vom piezoelektrischen Typ ist, und daß auf der Seite gegenüberliegend derjenigen, die gegen die Lagerung (8) gerichtet ist, eine Kammer (25, 26) ausgebildet ist, welche es jedem der Wandler erlaubt, zu vibrieren.

17. Vorrichtung gemäß Anspruch 16, dadurch gekennzeichnet, daß eine (25) der Kammern (25, 26) zum Wandler (9) hin im Innern des ersten Arms (17) des U-förmigen Körpers ausgebildet ist, während die andere Kammer (26), welche zum anderen Wandler (10) hin liegt, in einem Stützelement (19) ausgebildet ist, das den Wandler (10) trägt, der an dem zweiten Arm (18) des U-förmigen Körpers (7) fixiert ist.

18. Vorrichtung gemäß Anspruch 17, dadurch gekennzeichnet, daß das Element (19), welches den Wandler (10) trägt, in ein Loch (20) quer zu dem zweiten Arm (18) des U-förmigen Körpers (7) eingepreßt ist.

19. Vorrichtung gemäß einem der vorhergehenden Ansprüche, dadurch gekennzeichnet, daß die Lagerung (8) einen elliptischen Querschnitt hat, dessen große Achse mit der gemeinsamen Symmetrieachse für die Wandler-Geber (9) und -Empfänger (10) zusammenfällt.

**Claims**

1. A device (1) for detecting either the presence of gas in a liquid contained in a deformable and elastic vessel (2) or the lowering of the level of such a liquid below a predetermined line inside the vessel, comprising:

a hollow body (7) having a housing capable of receiving a portion (5) of the said vessel in a prearranged position in relation to the said predetermined line,

a pair of transducers, a transmitter (9) and a receiver (10) respectively of a predetermined signal, fixed to the said hollow body in positions on diametrically opposite sides facing each other, turned towards the said housing (8); the said receiver-transducer (10) being capable of cooperating with the said transmitter-transducer (9) and of emitting a signal responsive to the quantity of gaseous fluid interposed between the said transducers (9, 10),

characterized in that it comprises, moreover, a movable element (11) connected to the said hollow body (7) by elastic means (12) and capable of transmitting a radial thrust to the said portion (5) of the vessel (2), capable of deforming it and of thus producing its adhesion in opposite zones (13, 14) to the transducers (9, 10) without the inclusion of air.

2. A device according to Claim 1, characterized in that the said hollow body (7) is in essence U-shaped and carries the transmitter-transducer (9) and receiver-transducer (10) on opposite arms (17, 18), the movable element (11) being capable of closing the said vessel (2) inside the said hollow body (7).

3. A device according to Claim 2, characterized in that the said movable element (11) is in essence constituted by a lid (11) pivoting round a pin (36) situated at the end of the arm (17) of the said hollow body (7) and being capable of being fastened to the end of the other arm (18) by fastening means (46).

4. A device according to Claim 3, characterized in that the said pin 36 is supported in a manner allowing it to slide along the said first arm (17), by means of at least one rod (38) axially movable against the action of the said elastic means (12).

5. A device according to Claim 4, characterized in that the elastic means (12) comprise at least one helical spring coaxial with the rod (38) and capable of exerting an elastic thrust on the pin (36) tending to bring the said pin (36), and together with it, the said lid (11) nearer the surfaces opposite the said first and second arms (17, 18) of the said U-shaped body (7).

6. A device according to Claim 5, characterized in that the said spring (12) is accommodated inside a cylindrical chamber (41) arranged at the base of the said first arm (17).

7. A device according to any one of Claims 3 to 6, characterized in that the said fastening means are in essence constituted by a pivoting arm (46) provided at one end with a hook (45) capable of engaging a corresponding part (44) at the end of the said lid (11).

8. A device according to Claim 7, characterized in that the said hook (45) of the said pivoting arm (46) tapers and is capable of engaging in a tapered recess (48) presented towards the end of the said lid (11).

9. A device according to Claim 8, characterized in that the said hook (45) and the said housing (48) have in esence trapezoidal shapes.

10. A device according to any one of Claims 7 to 9, characterized in that the said arm (46) pivots round a pin (47) carried by the said second arm (18) of the said U-shaped hollow body (7).

11. A device according to Claim 10, characterized in that the said arm (46) pivots round the pin (47) against the action of elastic means (50).

12. A device according to Claim 11, characterized in that the elastic means (50) are in essence interposed between the end (49) of the pivoting arm (46) which on the one hand, is remote from the hook (45) and the opposite surface of the said second arm (18) on the other hand.

13. A device according to Claim 12, characterized in that the said end (49) of the pivoting arm has a boss (52) extending towards the outside and provided with a dish (53) delimiting a zone for controlling the rotation of the pivoting arm (46).

14. A device according to any one of the preceding claims, characterized in that the surface of each transducer, the transmitter transducer (9) and the receiver transducer (10), directed towards the housing (8) is covered by a layer (23, 24) of a protective material.

15. A device according to Claim 14, characterized in that the material in question is an epoxy resin.

16. A device according to any one of the preceding claims, characterized in that each transducer (9, 10) is of the piezoelectric type, and in that on the opposite to that directed towards the housing (8), it faces a chamber (25, 26), allowing each one of the said transducers to vibrate.

17. A device according to Claim 16, characterized in that one, (25), of the said chambers (25, 26) opposite the transducer (9) is arranged inside the said first arm (17) of the said U-shaped body, whilst the other chamber (26) opposite the other transducer (10) is arranged in a supporting element (19) carrying the said transducer (10) fixed to the said second arm of the said U-shaped body (7).

18. A device according to Claim 17, characterized in that the said element (19) supporting the said transducer (10) is force-fitted in a hole (20), transverse to the said second arm (18) of the said U-shaped body (7).

19. A device according to any one of the preceding claims, characterized in that the said housing (8) has an elliptical cross-section whose major axis coincides with the axis of symmetry common to the said transmitter and receiver transducers (9) and (10).

# Fig.1.

EP 0 181 272 B1

## Fig.2.

## Fig.4.

2

# EP 0 181 272 B1

## Fig.3.